# EUROPEAN PATENT SPECIFICATION

(11) **EP 2 394 598 B1**
(45) Date of publication and mention of the grant of the patent: **07.08.2013**
(21) Application number: 11004685.1
(22) Date of filing: 08.06.2011
(51) Int. Cl.: A61B 18/18, A61B 18/00, A61B 17/00

(54) **Energy applicator temperature monitoring for assessing ablation size**
Überwachung der Temperatur eines Energieapplikators zur Beurteilung der Ablationsgröße
Surveillance de la température d'applicateur d'énergie pour évaluer la taille d'ablation

(30) Priority: 09.06.2010 US 353135 P
(43) Date of publication of application: 14.12.2011
(73) Proprietor: Covidien LP, Mansfield, MA 02048 (US)
(72) Inventor: Coe, Jonathan, A., Menlo Park CA 94025 (US); Ladtkow, Casey, M., Erie, CO 80516 (US)
(74) Representative: Beyer, Andreas

(56) References cited:
- EP-A2- 1 462 065
- US-A1- 2008 275 440
- US-A1- 2010 082 022
- US-B1- 6 500 175

## Description

### BACKGROUND

### 1. Technical Field

The present disclosure relates to electrosurgical devices suitable for use in tissue ablation applications and, more particularly, to systems for directing energy to tissue and assessing ablation size as a function of temperature information associated with an energy applicator.

### 2. Discussion of Related Art

Treatment of certain diseases requires the destruction of malignant tissue growths, e.g., tumors. Electromagnetic radiation can be used to heat and destroy tumor cells. Treatment may involve inserting ablation probes into tissues where cancerous tumors have been identified. Once the probes are positioned, electromagnetic energy is passed through the probes into surrounding tissue.

In the treatment of diseases such as cancer, certain types of tumor cells have been found to denature at elevated temperatures that are slightly lower than temperatures normally injurious to healthy cells. Known treatment methods, such as hyperthermia therapy, heat diseased cells to temperatures above 41 ° C while maintaining adjacent healthy cells below the temperature at which irreversible cell destruction occurs. These methods involve applying electromagnetic radiation to heat, ablate and/or coagulate tissue. Microwave energy is sometimes utilized to perform these methods. Other procedures utilizing electromagnetic radiation to heat tissue also include coagulation, cutting and/or ablation of tissue. Many procedures and types of devices utilizing electromagnetic radiation to heat tissue have been developed.

The extent of tissue heating may depend on several factors including the rate at which energy is absorbed by, or dissipated in, the tissue under treatment. In treatment methods utilizing electromagnetic radiation, such as hyperthermia therapy, the transference or dispersion of heat generally may occur by mechanisms of radiation, conduction, and convection. Biological effects that result from heating of tissue by electromagnetic energy are often referred to as "thermal" effects.

One aspect of tumor ablation is the ablative margin, or the minimum distance between the surface of the tumor and the surface of the encompassing zone of ablated or thermally-necrosed tissue. Ablative margin is often difficult to assess, and ablation size and geometry may be used as a proxy.

Unfortunately, current methods for assessing ablation size are limited. Physicians typically utilize computed tomography (CT) or other imaging modality to assess the ablation size, which requires an interruption of treatment and may subject both the patient and the imaging personnel to a radiation dosage from the medical imaging. Thermometry probes may also be used, relying on the established relationship between time, temperature and cellular necrosis. However, this approach requires additional hardware and a more invasive approach to surgery, as additional insertion sites are generally required for the thermometry probes.

Methods of assessing ablation size without the need for medical imaging or thermometry may help to ensure clinical safety. Tumor treatment methods that do not require imaging to assess ablation size may reduce the radiation risk to the patient from multiple CT scans and other imaging modalities.

US 2008/275440 A1 discloses an ablation device comprising a catheter supplied with energy and coolant by a generator. The catheter includes a shaft and a distal tip. A needle, operating as an electrode, can protrude from the shaft. It is possible to measure the decay of tissue temperature to characterize the size of lesion formed. Various types of thermocouples are disclosed.

US 6 500 175 B1 discloses an ablation device for ablating living tissue.

EP 2 008 604 A2 discloses an antenna ablation apparatus, including an electrode, a cooling medium conduit and a tip. Probes, made of metal materials, can be inserted in the electrode and advanced therefrom. Temperature sensors are coupled with the probes. The temperature sensors are spatially distributed at various points within the tissue mass.

US 2010/0820022 A1 discloses a microwave energy delivery device and temperature sensor assemblies. Each temperature sensor assembly has a plurality of temperature probes and a sensor spaced away from the antenna for measuring the temperature of tissue.

### SUMMARY

The present invention is defined in claim 1.

The present disclosure relates to an apparatus for assessing ablation size as a function of temperature information associated with an energy applicator including the initial step of positioning an energy applicator in tissue. The energy applicator includes a radiating section and a temperature sensor. The radiating section is operably coupled to an energy source. The apparatus is provided, configured and intended for delivering energy from the energy source through the radiating section to tissue. The apparatus is provided, configured and intended for causing cessation of energy delivery through the radiating section to tissue for a predetermined time interval, monitoring the temperature sensor for at least a portion of the predetermined time interval to obtain temperature information associated with the energy applicator during the at least a portion of the predetermined time interval, and evaluating the temperature information to assess ablation size.

The present disclosure also relates to an apparatus provided, configured and intended for directing energy to tissue. The apparatus includesan energy applicator including a radiating section operably coupled to an electrosurgical power generating source and a temperature sensor operably coupled to the radiating section. The the energy applicator is provided, configured and intended for being positioned in tissue and provided, configured and intended for delivering energy from the electrosurgical power generating source through the radiating section to tissue for a first predetermined time interval. The apparatus also includes means provided, configured and intended for monitoring the temperature sensor for a second predetermined time interval to obtain temperature information associated with the radiating section. The apparatus further includes means provided, configured and intended for evaluating the temperature information to estimate ablation size, and determining one or more operating parameters associated with the electrosurgical power generating source based on the estimated ablation size.

The invention is as defined in the appended claims.

### BRIEF DESCRIPTION OF THE DRAWINGS

Objects and features of the presently disclosed systems for directing energy to tissue and the presently disclosed apparatus includes means provided, configured and intended for assessing ablation size as a function of temperature information associated with an energy applicator will become apparent to those of ordinary skill in the art when descriptions of various embodiments thereof are read with reference to the accompanying drawings, of which:

FIG. 1 is a schematic illustration of a system including an energy applicator array positioned for the delivery of energy to a targeted tissue area according to an embodiment of the present disclosure;

FIG. 2 is a block and sectional diagram of an ablation system including an energy applicator according to an embodiment of the present disclosure;

FIG. 3 shows a post-ablation thermal profile plot according to an embodiment of the present disclosure;

FIG. 4 shows a fitted line plot of active-electrode temperature monitoring data recorded at the active electrode tip of an energy applicator according to an embodiment of the present disclosure;

FIG. 5 is a flowchart illustrating a method of assessing ablation size as a function of temperature information associated with an energy applicator; and

FIG. 6 is a flowchart illustrating directing energy to tissue utilizing an apparatus including means according to an embodiment of the present disclosure.

### DETAILED DESCRIPTION

Hereinafter, embodiments of the system for directing energy to tissue and embodiments of the apparatus provided, configured and intended for assessing ablation size as a function of temperature information associated with an energy applicator of the present disclosure are described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the apparatus, or component thereof, closer to the user and the term "distal" refers to that portion of the apparatus, or component thereof, farther from the user.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)". For the purposes of this description, a phrase in the form "at least one of A, B, or C" means "(A), (B), (C), (A and B), (A and C), (B and C), or (A, B and C)".

Electromagnetic energy is generally classified by increasing energy or decreasing wavelength into radio waves, microwaves, infrared, visible light, ultraviolet, X-rays and gamma-rays. As it is used in this description, "microwave" generally refers to electromagnetic waves in the frequency range of 300 megahertz (MHz) (3 x 10⁸ cycles/second) to 300 gigahertz (GHz) (3 x **10¹¹** cycles/second). As it is used in this description, "ablation procedure" generally refers to any ablation procedure, such as, for example, microwave ablation, radiofrequency (RF) ablation, or microwave or RF ablation-assisted resection.

As it is used in this description, "energy applicator" generally refers to any device that can be used to transfer energy from a power generating source, such as a microwave or RF electrosurgical generator, to tissue. As it is used in this description, "transmission line" generally refers to any transmission medium that can be used for the propagation of signals from one point to another. As it is used in this description, "fluid" generally refers to a liquid, a gas or both.

Various embodiments of the present disclosure provide systems for directing energy to tissue. Various embodiments of the present disclosure provide systems capable of energy applicator temperature monitoring and for assessing ablation size (also referred to herein as "ablation volume") as a function of temperature information associated with an energy applicator. Embodiments may be implemented using electromagnetic radiation at microwave frequencies or at other frequencies. An electromagnetic energy delivery device including an energy applicator array, according to various embodiments, is designed and configured to operate between about 300 MHz and about 10 GHz.

Various embodiments of the presently disclosed electrosurgical system including an energy applicator, or energy applicator array, are suitable for microwave ablation and for use to pre-coagulate tissue for microwave ablation-assisted surgical resection. In addition, although the following description describes the provision and the use of a dipole microwave antenna and RF electrodes, the teachings of the present disclosure may also apply to a monopole, helical, or other suitable type of microwave antenna.

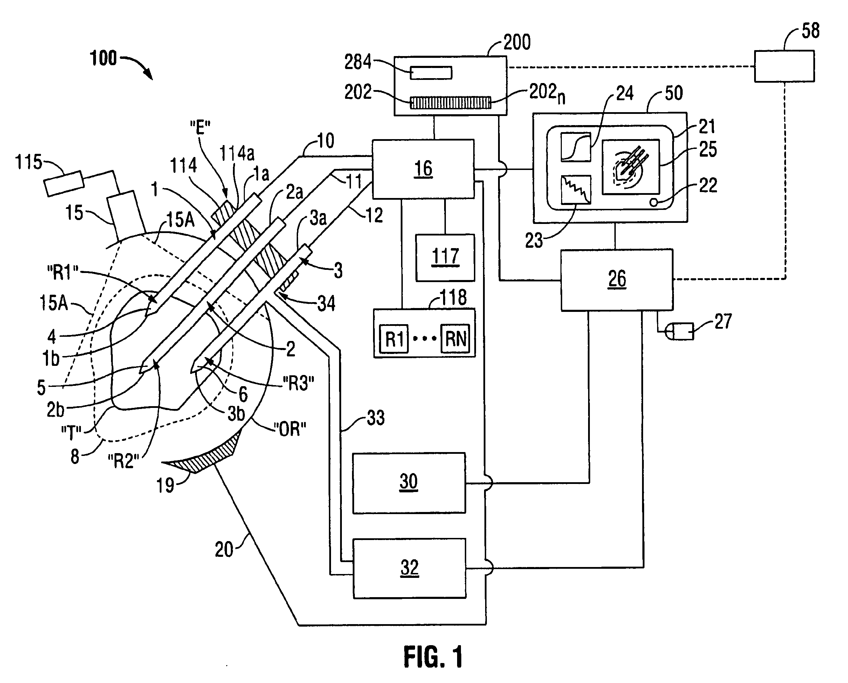

An electrosurgical system 100 according to an embodiment of the present disclosure is shown in FIG. 1 and includes an electromagnetic energy delivery device or energy applicator array "E". Energy applicator array "E" may include one or more energy applicators or probes. Probe thickness may be minimized, e.g., to reduce trauma to the surgical site and facilitate accurate probe placement to allow surgeons to treat target tissue with minimal damage to surrounding healthy tissue. In some embodiments, the energy applicator array "E" includes a plurality of probes. The probes may have similar or different diameters, may extend to equal or different lengths, and may have a distal end with a tapered tip. In some embodiments, the one or more probes may be provided with a coolant chamber. The probe(s) may be integrally associated with a hub (e.g., hub 34 shown in FIG. 1) that provides electrical and/or coolant connections to the probe(s). Additionally, or alternatively, the probe(s) may include coolant inflow and outflow ports to facilitate the flow of coolant into, and out of, the coolant chamber. Examples of coolant chamber and coolant inflow and outflow port embodiments are disclosed in commonly assigned U.S. Patent Application Serial No. 12/401,268 filed on March 10, 2009, entitled "COOLED DIELECTRICALLY BUFFERED MICROWAVE DIPOLE ANTENNA", and U.S. Patent No. 7,311,703, entitled "DEVICES AND METHODS FOR COOLING MICROWAVE ANTENNAS".

In the embodiment shown in FIG. 1, the energy applicator array "E" includes three energy applicators or probes 1, 2 and 3 having different lengths and arranged substantially parallel to each other. Probes 1, 2 and 3 generally include a radiating section "R1", "R2" and "R3", respectively, operably connected by a feedline (or shaft) 1a, 2a and 3a, respectively, to an electrosurgical power generating source 16, e.g., a microwave or RF electrosurgical generator. Transmission lines 10, 11 and 12 may be provided to electrically couple the feedlines 1 a, 2a and 3a, respectively, to the electrosurgical power generating source 16.

Located at the distal end of each probe 1, 2 and 3 is a tip portion 1b, 2b and 3b, respectively, which may be configured to be inserted into an organ "OR" of a human body or any other body tissue. Tip portion 1 b, 2b and 3b may terminate in a sharp tip to allow for insertion into tissue with minimal resistance. Tip portion 1b, 2b and 3b may include other shapes, such as, for example, a tip that is rounded, flat, square, hexagonal, or cylindroconical. The shape, size and number of probes of the energy applicator array "E" may be varied from the configuration depicted in FIG. 1.

Probes 1, 2 and 3 include a temperature sensor 4, 5 and 6, respectively, disposed within or in contact with the tip portion 1 b, 2b and 3b, respectively. Temperature sensors 4, 5 and 6 may be for example, a thermocouple, a thermistor, or any other type of temperature sensing device capable of sending a signal indicative of a temperature of a tip portion 1b, 2b and 3b to a processor unit 26.

An electrosurgical system 100 according to embodiments of the present disclosure includes a user interface 50 that preferably includes a display device 21, such as without limitation a flat panel graphic LCD (liquid crystal display), adapted to visually display one or more user interface elements (e.g., 23, 24 and 25 shown in FIG. 1). In an embodiment, the display device 21 includes touchscreen capability, e.g., the ability to receive user input through direct physical interaction with the display device 21, e.g., by contacting the display panel of the display device 21 with a stylus or fingertip. A user interface element (e.g., 23, 24 and/or 25 shown in FIG. 1) preferably has a corresponding active region, such that, by touching the display panel within the active region associated with the user interface element, an input associated with the user interface element is received by the user interface 50.

The user interface 50 preferably includes additionally, or alternatively, one or more controls 22 that may include without limitation a switch (e.g., pushbutton switch, toggle switch, slide switch) and/or a continuous actuator (e.g., rotary or linear potentiometer, rotary or linear encoder). In an embodiment, a control 22 has a dedicated function, e.g., display contrast, power on/off, and the like. Control 22 may also have a function that may vary in accordance with an operational mode of the electrosurgical system 100. A user interface element (e.g., 23 shown in FIG. 1) may be provided to indicate the function of the control 22. Control 22 may also include an indicator, such as an illuminated indicator, e.g., a single- or variably-colored LED (light emitting diode) indicator.

In some embodiments, the electrosurgical power generating source 16 is configured to provide microwave energy at an operational frequency from about 300 MHz to about 2500 MHz. In other embodiments, the electrosurgical power generating source 16 is configured to provide microwave energy at an operational frequency from about 300 MHz to about 10 GHz. Electrosurgical power generating source 16 may be configured to provide various frequencies of electromagnetic energy.

Feedlines 1a, 2a and 3a may be formed from a suitable flexible, semi-rigid or rigid microwave conductive cable, and may connect directly to an electrosurgical power generating source 16. Feedlines 1 a, 2a and 3a may have a variable length from a proximal end of the radiating sections "R1", "R2" and "R3", respectively, to a distal end of the transmission lines 10, 11 and 12, respectively, ranging from a length of about one inch to about twelve inches. Feedlines 1 a, 2a and 3a may be made of stainless steel, which generally offers the strength required to puncture tissue and/or skin. Feedlines 1a, 2a and 3a preferably include an inner conductor, a dielectric material coaxially surrounding the inner conductor, and an outer conductor coaxially surrounding the dielectric material. Radiating sections "R1", "R2" and "R3" are preferably formed from a portion of the inner conductor that extends distal of the feedlines 1a, 2a and 3a, respectively, into the radiating sections "R1", "R2" and "R3", respectively. In one embodiment, the feedlines 1a, 2a and 3a are cooled by fluid, e.g., saline, water or other suitable coolant fluid, to improve power handling, and may include a stainless steel catheter. Transmission lines 10, 11 and 12 may additionally, or alternatively, provide a conduit configured to provide coolant fluid from a coolant source 32 to the energy applicator array "E".

As shown in FIG. 1, the electrosurgical system 100 preferably further includes a reference electrode 19 (also referred to herein as a "return" electrode). Return electrode 19 may be electrically coupled via a transmission line 20 to the power generating source 16. During a procedure, the return electrode 19 may be positioned in contact with the skin of the patient or a surface of the organ "OR". Upon activating the energy applicator array "E", the return electrode 19 and the transmission line 20 may serve as a return current path for the current flowing from the power generating source 16 through the probes 1, 2 and 3.

During microwave ablation, e.g., using the electrosurgical system 100, the energy applicator array "E" is inserted into or placed adjacent to tissue and microwave energy is supplied thereto. Ultrasound or computed tomography (CT) guidance may be used to accurately guide the energy applicator array "E" into the area of tissue to be treated. Probes 1, 2 and 3 may be placed percutaneously or surgically, e.g., using conventional surgical techniques. The length of time that microwave energy is to be applied is prefererably determined prior or during applying the microwave energy. Application duration may depend on a variety of factors such as energy applicator design, number of energy applicators used simultaneously, tumor size and location, and whether the tumor was a secondary or primary cancer. The duration of microwave energy application using the energy applicator array "E" may depend on the progress of the heat distribution within the tissue area that is to be destroyed and/or the surrounding tissue.

FIG. 1 shows a targeted region including ablation targeted tissue represented in sectional view by the solid line "T". It may be desirable to ablate the targeted region "T" by fully engulfing the targeted region "T" in a volume of lethal heat elevation. Targeted region "T" may be, for example, a tumor that has been detected by a medical imaging system 30.

Medical imaging system 30, according to various embodiments, includes a scanner (e.g., 15 shown in FIG. 1) of any suitable imaging modality. Scanner 15 may be disposed in contact with the organ "OR" to provide image data. As an illustrative example, the two dashed lines 15A in FIG. 1 bound a region for examination by the scanner 15, e.g., a real-time ultrasonic scanner. In some embodiments, the medical imaging system 30 may be a multi-modal imaging system capable of scanning using different modalities.

Electrosurgical system 100 includes a processor unit 26. Processor unit 26 may include any type of computing device, computational circuit, or any type of processor or processing circuit capable of executing a series of instructions that are stored in a memory (not shown) associated with the processor unit 26. Processor unit 26 may be adapted to run an operating system platform and application programs. Processor unit 26 may receive user inputs from a keyboard (not shown), a pointing device 27, e.g., a mouse, joystick or trackball, and/or other device communicatively coupled to the processor unit 26.

In FIG. 1, the dashed line 8 surrounding the targeted region "T" represents the ablation isotherm in a sectional view through the organ "OR". Such an ablation isotherm may be that of the surface achieving possible temperatures of approximately 50° C or greater. The shape and size of the ablation isotherm volume, as illustrated by the dashed line 8, may be influenced by a variety of factors including the configuration of the energy applicator array "E", the geometry of the radiating sections "R1", "R2" and "R3", cooling of the probes 1, 2 and 3, ablation time and wattage, and tissue characteristics, e.g., tissue impedance.

Processor unit 26 may be connected to one or more display devices (e.g., 21 shown in FIG. 1) for displaying output from the processor unit 26, which may be used by the clinician to visualize the targeted region "T" and/or the ablation isotherm volume 8 during a procedure, e.g., an ablation procedure. In embodiments, real-time data and/or near real-time data acquired from CT scan, ultrasound, or MRI (or other scanning modality) may be outputted from the processor unit 26 to one or more display devices.

Electrosurgical system 100 may include a library 200 including a plurality of thermal profiles or overlays 202-202ₙ. As it is used in this description, "library" generally refers to any repository, databank, database, cache, storage unit and the like. Each of the overlays 202-202ₙ may include a thermal profile that is characteristic of and/or specific to a particular energy applicator design, particular energy applicator array, and/or exposure time. Library 200 may include a database 284 that is configured to store and retrieve energy applicator data, e.g., parameters associated with one or energy applicators (e.g., 1, 2 and 3 shown in FIG. 1) and/or one or more energy applicator arrays (e.g., "E" shown in FIG. 1). Ablation pattern topology may be included in the database 284, e.g., a wireframe model of an energy applicator array (e.g., 25 shown in FIG. 1) and/or a representation of a radiation pattern associated therewith. Library 200 may be in communicatively associated with a picture archiving and communication system (PACS) database (shown generally as 58 in FIG. 1). Processor unit 26 may be communicatively associated with the PACS database 58.

Images and/or non-graphical data stored in the library 200, and/or retrievable from the PACS database 58, may be used to configure the electrosurgical system 100 and/or control operations thereof. Data associated with an estimated ablation size may be stored in a database (e.g., 284 shown in FIG. 1) prior to and/or during a procedure.

One or more parameters of an estimated ablation size in accordance with the present disclosure may be used as a feedback tool to control an instrument's and/or clinician's motion, e.g., to avoid ablating critical structures, such as large vessels, healthy organs or vital membrane barriers. Examples of parameters of an estimated ablation size include without limitation volume, length, diameter, minimum diameter, maximum diameter and centroid. Images and/or other information displayed on the display device 21 of the user interface 50 may be used to better visualize and understand how to achieve more optimized results during thermal treatment of tissue, such as, for example, ablation of tissue, tumors and cancer cells.

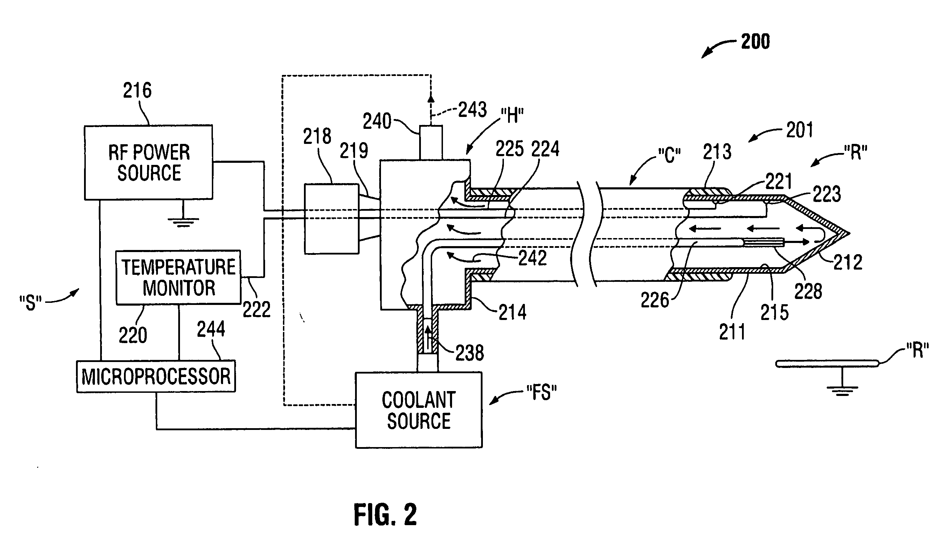

FIG. 2 shows an ablation system 200 including an energy applicator 201 according to an embodiment of the present disclosure. Energy applicator 201 includes an elongated shaft or cannula body "C" for positioning in tissue, e.g., percutaneously or intraoperatively into an open wound site. In an embodiment, the cannula body "C" is integral with a head or hub element "H" coupled to remote support components, collectively designated "S".

Cannula body "C" includes an elongated ablative electrode 211 formed of conductive material, e.g. metal such as stainless steel, titanium, etc. Electrode 211 may include a substantially hollow tubular body sized in length and diameter to fit within the cannula body "C". At the distal end of the cannula body "C", the electrode 211 defines a tip 212. In operation when using an RF power supply 216, electrical current spreads from the tip 212 to pass through the surrounding tissue causing the tissue to heat up.

Electrode 211 carries an insulative coating 213 over a portion of its length for selectively preventing the flow of electrical current from the shaft 215 of electrode 211 into surrounding tissue. lnsulative coating 213 shields the intervening tissue from RF current, so that tissue along the length of the shaft 215 is not substantially heated except by the heating effect from the exposed tip 212.

The proximal end of the electrode 211 is integral with an enlarged housing 214 of the hub "H", which carries electrical and coolant connections as described below. In the portion disposed outside the patient's body, the housing 214 is of cylindrical configuration, defining ports for connections to the support components "S", e.g., electrical and fluid couplings. Housing 214 may be integral with the electrode 211, formed of metal, or it may constitute a separate subassembly as described below. Housing 214 may be formed of plastic, and may accommodate separate electrical connections. In that regard, a plastic housing 214 is amenable to low artifact imaging by X-rays CT, MRI, etc. as may be desirable in some situations.

The housing 214 mates with a block 218 defining a luer taper lock 219 sealing the block 218 to the housing 214. Connection to a regulated RF power source 216 may take the form of a standard cable connector, a leader wire, a jack-type contact or other designs. The temperature-sensing and radiofrequency electrical connections can be made through the housing 214 and extend to the region of the tip 212, where an RF line 225 is connected by junction 221, e.g., a weld, braze, or other secure electrical connection. In embodiments, sensor lines 224 extending to a thermo-sensor 223, e.g., a thermistor, or a thermocouple, or any other type of temperature sensing device capable of sending a signal indicative of a temperature. Thermo-sensor 223 may be fused or in thermal contact with the wall of the tip 212 to sense the temperature of the tip 212.

RF power source 216 may be referenced to reference potential, as illustrated in FIG. 2, and coupled through the block 218 affixed to the hub "H". In embodiments, the RF power source 216 provides RF voltage through the block 218 with an electrical connection to the electrode 211 as indicated by the line 225, to the connection junction 221. RF power source 216 may take the form of an RF generator as exemplified by the RFG-3C RF Lesion Generator System available from Radionics, Inc., Burlington, Mass.

As indicated above, when the ablation electrode 211 is in a patient's body, an electrical circuit is completed through the body to a reference or dispersive electrode R (symbolically represented in FIG. 2) that is connected elsewhere to the body. Energy transmitted from the RF power source 216 heats body tissue by current from the tip 212. In that regard, a temperature monitor 220 may be electrically connected by lines 222 and 224 to a temperature sensor 223 disposed within or contacting the tip 212. Temperature sensor 223 may be a thermocouple, thermistor, or other temperature sensing device. In an embodiment, the sensor 223 is connected to the tip 212. The sensed temperature may be utilized to control either or both of the flow of RF energy or the flow of coolant to attain the desired ablation while maintaining the maximum temperature substantially below a predetermined temperature, e.g., 100° C. A plurality of sensors could be utilized including units extending outside the tip 212 to measure temperatures at various locations in the proximity of the tip 212. Examples of temperature monitoring devices that may suitably be used as the temperature monitor 220 may include the TC thermocouple temperature monitoring devices available from Radionics, Inc., Burlington, Massachusetts.

In accordance herewith, temperatures at, or near the tip 212 (e.g., manifest by the temperature monitor 220) may be controlled by controlling the flow of coolant fluid through the ablation electrode 211. In this manner, the temperature of the surface area of the tip 212 in contact with tissue is controllable. In an embodiment, fluid from a fluid source "FS" is carried the length of the ablation electrode 211 through a tube 226 extending from the housing 214 to the distal end of the electrode 211 terminating in an open end 228 at the tip 212. At the proximal end of the electrode 211, within the housing 214, the tube 226 is connected to receive coolant fluid. Fluid flow may be regulated in accordance with the sensed temperature sensed at the tip 212, allowing increased flow of RF energy.

The fluid coolant may take the form of water or saline for the convection removal of heat from the tip 212. A reservoir or source unit for supplying coolant fluid may be a large reservoir of cooled water, saline or other fluid. As a simplistic example, a tank of water with ice cubes can function to maintain the coolant at a temperature of approximately 0° C. As another example, the fluid source "FS" could incorporate a peristaltic pump or other fluid pump, or could merely be a gravity feed for supplying fluid from a bag or rigid tank.

Flow away from the tip 212 is back to the hub "H" to exit the hub "H" through an exit port 240 as illustrated by arrows 242 and 243. The ports may take the form of simple couplings, rigid units or may include flexible tubular couplings to reduce torque transmission to the electrode 211. The coolant flow members may take the form of PVC tubes with plastic luer connectors for ease of use.

As a result of the coolant flow, the interior of the electrode 211, e.g., the electrode tip 212, can be held to a temperature near that of the fluid source "FS". The coolant can circulate in a closed system as illustrated in FIG. 2. In some situations, it may be desirable to reverse the direction of fluid flow from that depicted in the FIG. 2. Coordinated operation involving RF heating along with the cooling may be accomplished by a microprocessor 244. In that regard, the microprocessor 244 is coupled to the RF power source 216, the temperature monitor 220 and the fluid source "FS" to receive data on flow rates and temperatures and exercise control. An integrated operation may be provided with feedback from the temperature monitor 20 in a controlled format and various functions can be concurrently accomplished. In embodiments, facilitated by the cooling, the ablation electrode 211 may be moderated, changed, controlled or stabilized. Such controlled operation may effectively reduce the temperature of tissue near the tip 212 to accomplish an equilibrium temperature distribution tailored to the size of the targeted tumor.

The temperature distribution in the tissue near the tip 212 generally depends on the RF current from the radiating section "R" and/or tip 212 and on the temperature of the tissue which is adjacent to the radiating section "R" and/or tip 212. The tip temperature can be controlled to approach the temperature of the fluid from the source "FS". In this manner, a thermal boundary condition may be established, holding the temperature of the tissue near the radiating section "R" and/or tip 212 to approximately the temperature of the tip itself, e.g., the temperature of the coolant fluid inside the tip 212. Accordingly, by temperature control, a defined temperature is preferably imposed at the boundary of the electrode radiating section "R" and/or tip 212 which can be somewhat independent of the RF heating process and may dramatically modify the temperature distribution in the tissue.

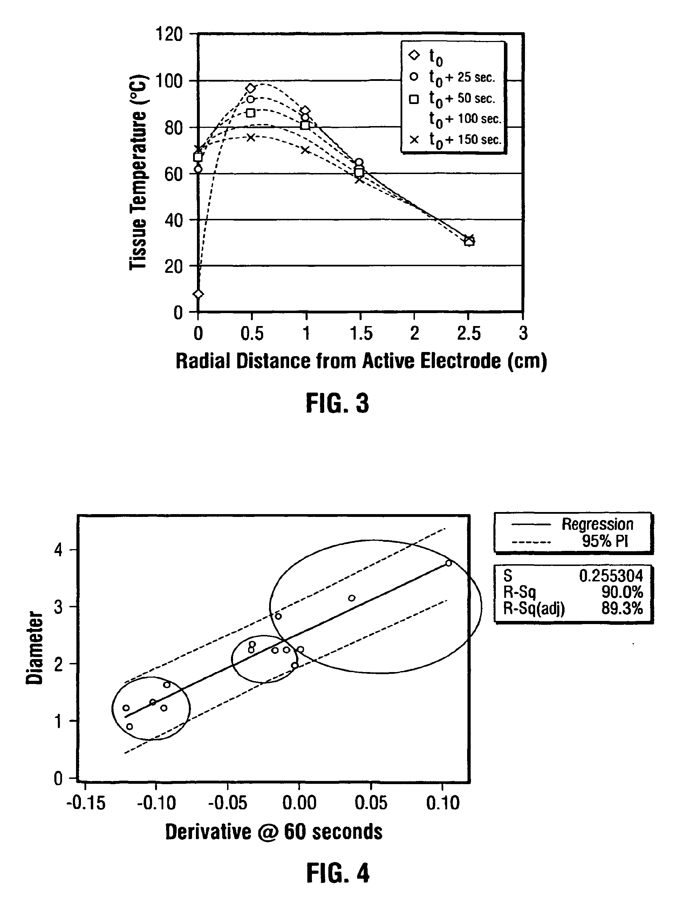

FIG. 3 shows a post-ablation thermal profile plot. The plot shows five different curves corresponding to tissue temperature (°C) in relation to radial distance from an active electrode (cm) at time equal to to, t₀+25 sec., t₀+50 sec., t₀+100 sec., and t₀+150 sec.

FIG. 4 shows a fitted line plot of active-electrode temperature monitoring data recorded at the active electrode tip of an energy applicator according to an embodiment of the present disclosure.

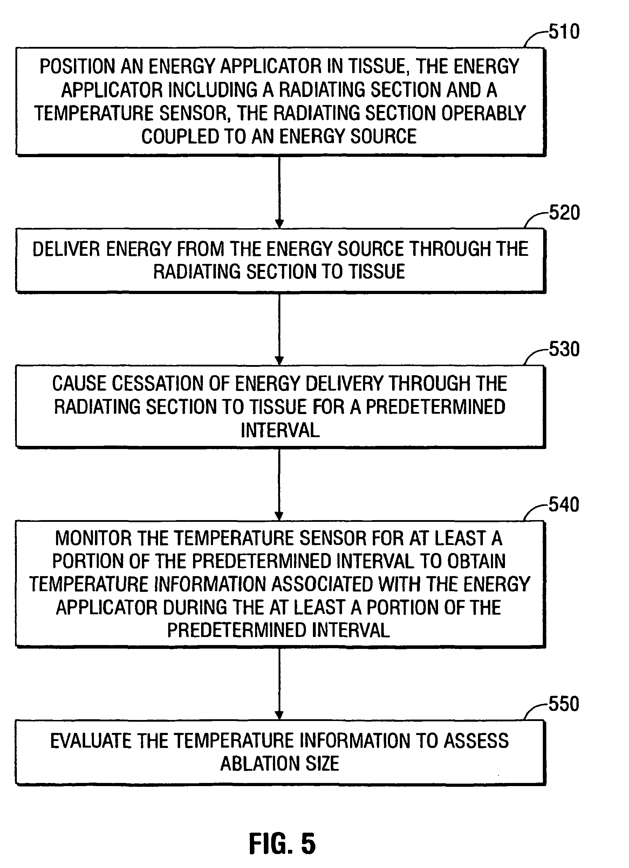

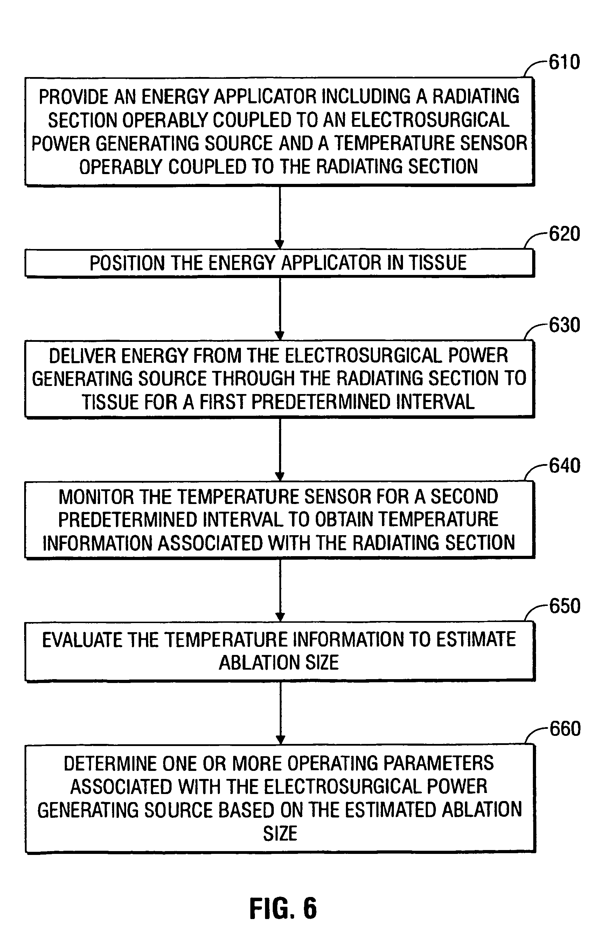

Hereinafter, an apparatus according tothepresent disclosure if assessing ablation size as a function of temperature information associated with an energy applicator is described with reference to FIG. 5 and an apparatus for directing energy to tissue is described with reference to FIG. 6. It is to be understood that the apparatus provided herein may be used in combination and in a different ways than presented herein without departing from the scope of the disclosure.

FIG. 5 is a flowchart illustrating the use of the apparatus for assessing ablation size as a function of temperature information associated with an energy applicator according to an embodiment of the present disclosure. In step 510, an energy applicator (e.g., energy applicator 1 of the energy applicator array "E" shown in FIG. 1) is positioned in tissue "T". Energy applicator 1 includes a radiating section "R1" and a temperature sensor 4. In some embodiments, temperature sensor 4 is disposed within a distal tip portion 1b of the energy applicator 1. Radiating section "R1" is operably coupled to an energy source 16. Energy applicator 1 may include a feedline 1a electrically coupled between the radiating section "R1" and a transmission line 10 electrically coupled to the energy source 16.

In step 520, energy from the energy source 16 is delivered through the radiating section "R1" to tissue "T". Embodiments may be implemented using electromagnetic radiation at microwave frequencies or at other frequencies. In some embodiments, the energy source 16 is an electrosurgical power generating source operable to output energy.

In step 530, energy delivery through the radiating section "R1" to tissue "T" is caused to cease for a predetermined time interval. The predetermined time interval may be any suitable interval. In some embodiments, the predetermined time interval may be about one second to about three minutes. Causing cessation of energy delivery through the radiating section "R1" to tissue "T" for a predetermined time interval may include controlling one or more operating parameters associated with the electrosurgical power generating source.

In step 540, the temperature sensor 4 is monitored for at least a portion of the predetermined time interval to obtain temperature information associated with the energy applicator 1 during the at least a portion of the predetermined time interval. Temperature sensor 4 may be monitored peri-operatively or post-operatively.

In step 550, the temperature information, obtained in step 540, is evaluated to assess ablation size. In embodiments, the ablation size may be assessed as a function of one or more of the rate of change in temperature of the energy applicator 1, or portion thereof, e.g., tip portion thereof; the maximum temperature reached by the temperature sensor 4 and the time integral of the temperature sensor measurement.

FIG. 6 is a flowchart illustrating the use of the apparatus for directing energy to tissue according to an embodiment of the present disclosure.

In step 610, an energy applicator (e.g., 201 shown in FIG. 2) is provided. Energy applicator 201 includes a radiating section "R" operably coupled to an electrosurgical power generating source 216 and a temperature sensor 223 operably coupled to the radiating section "R". Electrosurgical power generating source 216 may be configured to provide various frequencies of electromagnetic energy.

In step 620, the energy applicator 201 is positioned in tissue. Energy applicator 201 may be inserted directly into tissue, inserted through a lumen, e.g., a vein, needle or catheter, placed into the body, or positioned in the body by other suitable methods. Ultrasound, computed tomography (CT) guidance, or other guidance may be used to accurately guide the energy applicator 201 into the area of tissue to be treated.

In step 630, energy from the electrosurgical power generating source 216 is delivered through the radiating section "R" to tissue for a first predetermined time interval. The first predetermined time interval may be any suitable interval, but an interval of between 30 seconds and two minutes has been shown to allow for an appreciable increase in tissue temperature.

In step 640, the temperature sensor 223 is monitored for a second predetermined time interval to obtain temperature information associated with the radiating section. This step may include receiving a signal indicative of a temperature of a tip portion 212 of the energy applicator 201, wherein said signal from the temperature sensor 223 is received by a processor unit 244.

In some embodiments, the first and second predetermined time intervals are successive time intervals. In some embodiments, monitoring the temperature sensor 223 for the second predetermined time interval, in step 640, includes controlling one or more operating parameters associated with the electrosurgical power generating source 216, e.g., to cause cessation of energy delivery through the radiating section "R" to tissue for the second predetermined time.

In step 650, the temperature information is evaluated to estimate ablation volume. This estimation may be determined from the assessed ablation size, for example, by using equations for the volume of a sphere or spheroid.

In step 660, one or more operating parameters associated with the electrosurgical power generating source 216 are determined based on one or more parameters of the estimated ablation volume. Examples of operating parameters associated with the electrosurgical power generating source 216 include without limitation temperature, impedance, power, current, voltage, mode of operation, and duration of application of electromagnetic energy. Examples of parameters of the estimated ablation volume include without limitation volume, length, diameter, minimum diameter, maximum diameter, and centroid.

In an embodiment, the presently disclosed apparatus for directing energy to tissue is provided, intended and used for delivering energy from the electrosurgical power generating source 216 through the radiating section "R" to tissue for a third predetermined time interval, monitoring the temperature sensor 223 for a fourth predetermined time interval to obtain supplementary temperature information associated with the radiating section, and evaluating the supplementary temperature information to assess a change in the estimated ablation volume. The apparatus for directing energy to tissue is provided, intended and used for repeating the above-mentioned additional steps any suitable number of times, wherein, at each iteration, one or more operating parameters associated with the electrosurgical power generating source 216 may be determined based on the change in the estimated ablation volume. In some embodiments, the first, second, third and fourth predetermined time intervals are successive time intervals.

The above-described systems may involve the use of temperature data associated with an energy applicator for assessing ablation size to facilitate planning and effective execution of a procedure, e.g., an ablation procedure.

The above-described systems and methods may involve the use of energy applicator temperature monitoring to assess ablation size as a function of the rate of change in temperature of the energy applicator. As described above, data including one or more parameters of an estimated ablation size may be stored in a PACS database, and the stored image data may be retrieved from the PACS database prior to and/or during a procedure, e.g., to facilitate planning and effective execution of an ablation procedure.

As described above, temperature data may be received from one or more energy applicators during a procedure, e.g., for use in assessing ablation size during the procedure. One or more operating parameters associated with an electrosurgical power generating source may be determined as a function of assessed ablation size during an ablation procedure.

According to various embodiments of the present disclosure, the ablation size, as determined by the above-described methods, may be used to control the positioning of an electrosurgical device (e.g., rotation of a energy applicator with a directional radiation pattern to avoid ablating sensitive structures, such as large vessels, healthy organs or vital membrane barriers) and/or control an electrosurgical power generating source operably associated with an energy applicator.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

## Claims

1. An apparatus (100; 200) including means provided, configured and intended for assessing ablation size as a function of temperature information associated with an energy applicator, comprising:
an energy applicator (1, 2, 3; 201) provided, configured and intended for being positioned in tissue, the energy applicator including a radiating section (R1, R2, R3; 211) and a temperature sensor (4, 5, 6; 223), the radiating section operably coupled to an energy source;
an electrosurgical power generating source (216);
the energy applicator being provided, configured and intended for delivering energy from the electrosurgical power generating source (216) through the radiating section to tissue (T);
the energy applicator being provided, configured and intended for causing cessation of energy delivery through the radiating section to tissue for a predetermined time interval;
means (220) provided, configured and intended for monitoring the temperature sensor (4, 5, 6; 223) for at least a portion of the predetermined time interval to obtain temperature information associated with the energy applicator during the at least a portion of the predetermined time interval; and
means provided, configured and intended for evaluating the temperature information to assess ablation size,
wherein the temperature sensor (4, 5, 6; 223) is provided, configured and intended for monitoring for at least a portion of the predetermined time interval to obtain temperature information associated with the energy applicator by receiving a signal from the temperature sensor indicative of a temperature of a tip portion (212) of the energy applicator (1, 2, 3; 201),
wherein a means is provided, configured and intended for evaluating the temperature information to assess ablation size by assessing ablation size as a function of the rate of change in temperature of the radiating section.

2. The apparatus for assessing ablation size as a function of temperature information associated with an energy applicator in accordance with claim 1, further comprising:
a means is provided, configured and intended for determining at least one operating parameter associated with the electrosurgical power generating source (216) based on at least one parameter of the ablation size.

3. The apparatus for assessing ablation size as a function of temperature information associated with an energy applicator in accordance with claim 2, wherein the means provided, configured and intended for determining at least one operating parameter associated with the electrosurgical power generating source (216) is configured to select the operating parameter from the group consisting of temperature, impedance, power, current, voltage, mode of operation, and duration of application of electromagnetic energy.

4. The apparatus for assessing ablation size as a function of temperature information associated with an energy applicator in accordance with claim 2, wherein the means provided, configured and intended for determining at least one parameter of the ablation size is configured to select from the group consisting of volume, length, diameter, minimum diameter, maximum diameter and centroid.

5. The apparatus for assessing ablation size as a function of temperature information associated with an energy applicator in accordance with claim 4, wherein means provided, configured and intended for causing cessation of energy delivery through the radiating section to tissue for a predetermined time interval includes means configured to control the electrosurgical power generating source (216).

6. The apparatus in accordance with claim 1, further comprising:
a means provided, configured and intended for monitoring the temperature sensor for a second predetermined time interval to obtain temperature information associated with the radiating section;
a means provided, configured and intended for evaluating the temperature information to estimate ablation volume; and
a means provided, configured and intended for determining at least one operating parameter associated with the electrosurgical power generating source (216) based on at least one parameter of the estimated ablation volume.

7. The apparatus in accordance with claim 6,
wherein the means provided, configured and intended for monitoring the temperature sensor for a second predetermined time interval to obtain temperature information associated with the radiating section includes a means provided, configured and intended for controlling the electrosurgical power generating source (216) to cause cessation of energy delivery through the radiating section to tissue for the second predetermined time.

8. The apparatus in accordance with claim 6,
wherein the at least one operating parameter associated with the electrosurgical power generating source (216) is selected from the group consisting of temperature, impedance, power, current, voltage, mode of operation, and duration of application of electromagnetic energy, and / or wherein the at least one parameter of the estimated ablation volume is selected from the group consisting of volume, length, diameter, minimum diameter, maximum diameter and centroid and/or wherein the second predetermined time interval is successive to the first predetermined time interval.

9. The apparatus in accordance with claim 6, further comprising:
means provided, configured and intended for delivering energy from the electrosurgical power generating source (216) through the radiating section to tissue for a third predetermined time interval;
means provided, configured and intended for monitoring the temperature sensor for a fourth predetermined time interval to obtain supplementary temperature information associated with the radiating section; and
means provided, configured and intended for evaluating the supplementary temperature information to assess a change in the estimated ablation volume.

10. The apparatus in accordance with claim 9, further comprising:
means provided, configured and intended for determining at least one operating parameter associated with the electrosurgical power generating source (216) based on the change in the estimated ablation volume.

11. The apparatus in accordance with claim 10,
wherein a means is provided, configured and intended for providing the third predetermined time interval successively to the second predetermined time interval.

12. The apparatus in accordance with claim 10, wherein a means is provided, configured and intended for providing the fourth predetermined time interval successively to the third predetermined time interval.

## Patentansprüche

1. Vorrichtung (100; 200) mit Mitteln, die dazu vorgesehen, angeordnet und gedacht sind, eine Ablationsgröße als Funktion einer mit einem Energieapplikator in Beziehung stehenden Temperaturinformation zu beurteilen, mit:
einem Energieapplikator (1, 2, 3; 201), der dazu vorgesehen, angeordnet und gedacht ist, in Gewebe angeordnet zu werden, wobei der Energieapplikator einen Abstrahlabschnitt (R1, R2, R3; 211) und einen Temperatursensor (4, 5, 6; 223) aufweist, wobei der Abstrahlabschnitt betriebsfähig mit einer Energiequelle verbunden ist,
einer elektrochirurgischen Leistungserzeugungsquelle (216),
wobei der Energieapplikator dazu vorgesehen, angeordnet und gedacht ist, Energie von der elektrochirurgischen Leistungserzeugungsquelle (216) durch den Abstrahlabschnitt an Gewebe (T) abzugeben,
wobei der Energieapplikator dazu vorgesehen, angeordnet und gedacht ist, eine Unterbrechung der Energieabgabe durch den Abstrahlabschnitt an das Gewebe für eine vorbestimmte Zeitdauer zu bewirken,
einer Einrichtung (220), die dazu vorgesehen, angeordnet und gedacht ist, den Temperatursensor (4, 5, 6; 223) für zumindest einen Teil der vorbestimmten Zeitdauer zu überwachen, um während des zumindest einen Teils der vorbestimmten Zeitdauer mit dem Energieapplikator in Beziehung stehende Temperaturinformation zu erhalten, und
einer Einrichtung, die dazu vorgesehen, angeordnet und gedacht ist, die Temperaturinformation auszuwerten, um die Ablationsgröße zu beurteilen,
wobei der Temperatursensor (4, 5, 6; 223) dazu vorgesehen, angeordnet und gedacht ist, für zumindest einen Teil der vorbestimmten Zeitdauer zu überwachen, um mit dem Energieapplikator in Beziehung stehende Temperaturinformation zu erhalten durch Empfangen eines Signals von dem Temperatursensor, welches eine Temperatur eines Spitzenabschnitts (212) des Energieapplikators (1, 2, 3; 201) angibt,
wobei eine Einrichtung dazu vorgesehen, angeordnet und gedacht ist, die Temperaturinformation zum Beurteilen der Ablationsgröße auszuwerten durch Beurteilen der Ablationsgröße als Funktion der Änderungsrate der Temperatur des Abstrahlabschnitts.

2. Vorrichtung zum Beurteilen einer Ablationsgröße als Funktion einer mit einem Energieapplikator in Beziehung stehenden Temperaturinformation nach Anspruch 1, ferner aufweisend:
eine Einrichtung, die dazu vorgesehen, angeordnet und gedacht ist, wenigstens einen mit der elektrochirurgischen Leistungserzeugungsquelle (216) in Beziehung stehenden Betriebsparameter basierend auf wenigstens einem Parameter der Ablationsgröße zu ermitteln.

3. Vorrichtung zum Beurteilen einer Ablationsgröße als Funktion einer mit einem Energieapplikator in Beziehung stehenden Temperaturinformation nach Anspruch 2, bei der die Einrichtung, welche dazu vorgesehen, angeordnet und gedacht ist, wenigstens einen mit der elektrochirurgischen Leistungserzeugungsquelle (216) in Beziehung stehenden Betriebsparameter zu ermitteln, dazu konfiguriert ist, den Betriebsparameter aus der Gruppe bestehend aus Temperatur, Impedanz, Leistung, Strom, Spannung, Betriebsart und Aufbringungsdauer elektromagnetischer Energie auszuwählen.

4. Vorrichtung zum Beurteilen einer Ablationsgröße als Funktion einer mit einem Energieapplikator in Beziehung stehenden Temperaturinformation nach Anspruch 2, bei der die Einrichtung, die dazu vorgesehen, angeordnet und gedacht ist, wenigstens einen Parameter der Ablationsgröße zu ermitteln, dazu konfiguriert ist, aus der Gruppe bestehend aus Volumen, Länge, Durchmesser, Minimaldurchmesser, Maximaldurchmesser und Schwerpunkt auszuwählen.

5. Vorrichtung zum Beurteilen einer Ablationsgröße als Funktion einer mit einem Energieapplikator in Beziehung stehenden Temperaturinformation nach Anspruch 4, bei der die Einrichtung, die dazu vorgesehen, angeordnet und gedacht ist, eine Unterbrechung einer Energieabgabe durch den Abstrahlabschnitt an Gewebe für eine vorbestimmte Zeitdauer zu bewirken, eine Einrichtung umfasst, die dazu konfiguriert ist, die elektrochirurgische Leistungserzeugungsquelle (216) zu steuern.

6. Vorrichtung nach Anspruch 1, ferner aufweisend:
eine Einrichtung, die dazu vorgesehen, angeordnet und gedacht ist, den Temperatursensor für eine zweite vorbestimmte Zeitdauer zu überwachen, um mit dem Abstrahlabschnitt in Beziehung stehende Temperaturinformation zu erhalten,
eine Einrichtung, die dazu vorgesehen, angeordnet und gedacht ist, die Temperaturinformation auszuwerten, um ein Ablationsvolumen abzuschätzen, und
eine Einrichtung, die dazu vorgesehen, angeordnet und gedacht ist, wenigstens einen mit der elektrochirurgischen Leistungserzeugungsquelle (216) in Beziehung stehenden Betriebsparameter basierend auf wenigstens einem Parameter des abgeschätzten Ablationsvolumens zu ermitteln.

7. Vorrichtung nach Anspruch 6, bei der die Einrichtung, die dazu vorgesehen, angeordnet und gedacht ist, den Temperatursensor für eine zweite vorbestimmte Zeitdauer zu überwachen, um mit dem Abstrahlabschnitt in Beziehung stehende Temperaturinformation zu erhalten, eine Einrichtung enthält, die dazu vorgesehen, angeordnet und gedacht ist, die elektrochirurgische Leistungserzeugungsquelle (216) zu steuern, um für die zweite vorbestimmte Zeit eine Unterbrechung der Energieabgabe durch den Abstrahlabschnitt an Gewebe zu bewirken.

8. Vorrichtung nach Anspruch 6, bei der der zumindest ein mit der elektrochirurgischen Leistungserzeugungsquelle (216) in Beziehung stehender Betriebsparameter ausgewählt ist aus der Gruppe bestehend aus Temperatur, Impedanz, Leistung, Strom, Spannung, Betriebsart und Aufbringungsdauer elektromagnetischer Energie und/oder bei der der zumindest eine Parameter des abgeschätzten Ablationsvolumens ausgewählt ist aus der Gruppe bestehend aus Volumen, Länge, Durchmesser, Minimaldurchmesser, Maximaldurchmesser und Schwerpunkt und/oder bei der die zweite vorbestimmte Zeitdauer auf die erste vorbestimmte Zeitdauer folgt.

9. Vorrichtung nach Anspruch 6, ferner aufweisend:
eine Einrichtung, die dazu vorgesehen, angeordnet und gedacht ist, Energie aus der elektrochirurgischen Leistungserzeugungsquelle (216) für eine dritte vorbestimmte Zeitdauer durch den Abstrahlabschnitt an Gewebe abzugeben,
eine Einrichtung, die dazu vorgesehen, angeordnet und gedacht ist, den Temperatursensor für eine vierte vorbestimmte Zeitdauer zu überwachen, um mit dem Abstrahlabschnitt in Beziehung stehende, zusätzliche Temperaturinformation zu erhalten, und
eine Einrichtung, die dazu vorgesehen, angeordnet und gedacht ist, die zusätzliche Temperaturinformation auszuwerten, um eine Änderung des abgeschätzten Ablationsvolumens festzustellen.

10. Vorrichtung nach Anspruch 9, ferner aufweisend:
eine Einrichtung, die dazu vorgesehen, angeordnet und gedacht ist, zumindest einen mit der elektrochirurgischen Leistungserzeugungsquelle (216) in Beziehung stehenden Betriebsparameter basierend auf der Änderung des abgeschätzten Ablationsvolumens zu ermitteln.

11. Vorrichtung nach Anspruch 10, bei der eine Einrichtung vorgesehen, angeordnet und dazu gedacht ist, die dritte vorbestimmte Zeitdauer auf die zweite vorbestimmte Zeitdauer folgend bereitzustellen.

12. Vorrichtung nach Anspruch 10, bei der eine Einrichtung vorgesehen, angeordnet und dazu gedacht ist, die vierte vorbestimmte Zeitdauer auf die dritte vorbestimmte Zeitdauer folgend bereitzustellen.

## Revendications

1. Appareil (100 ; 200) incluant un moyen prévu pour, configuré pour et destiné à évaluer une taille d'ablation comme une fonction d'une information de température associée avec un applicateur d'énergie, comprenant :
un applicateur d'énergie (1, 2, 3 ; 201) prévu pour, configuré pour et destiné à être positionné dans un tissu, l'applicateur d'énergie incluant une section de rayonnement (R1, R2, R3 ; 211) et un capteur de température (4, 5, 6 ; 223), la section de rayonnement opérationnellement couplée à une source d'énergie ;
une source de génération d'alimentation électrochirurgicale (216) ;
l'applicateur d'énergie étant prévu pour, configuré pour et destiné à délivrer une énergie à partir de la source de génération d'alimentation électrochirurgicale (216) par l'intermédiaire de la section de rayonnement jusqu'à un tissu (T) ;
l'applicateur d'énergie étant prévu pour, configuré pour et destiné à causer une interruption de la délivrance d'énergie par l'intermédiaire de la section de rayonnement au tissu sur un intervalle de temps prédéterminé ;
un moyen (220) prévu pour, configuré pour et destiné à surveiller le capteur de température (4, 5, 6 ; 223) sur au moins une partie de l'intervalle de temps prédéterminé pour obtenir une information de température associée avec l'applicateur d'énergie pendant l'au moins une partie de l'intervalle de temps prédéterminé ; et
un moyen prévu pour, configuré pour et destiné à évaluer l'information de température pour évaluer une taille d'ablation,
dans lequel le capteur de température (4, 5, 6 ; 223) est prévu pour, configuré pour et destiné à surveiller sur au moins une partie de l'intervalle de temps prédéterminé pour obtenir une information de température associée avec l'applicateur d'énergie en recevant un signal du capteur de température indicateur d'une température d'une partie de pointe (212) de l'applicateur d'énergie (1, 2, 3 ; 201),
dans lequel un moyen est prévu pour, configuré pour et destiné à évaluer l'information de température pour évaluer une taille d'ablation en évaluant une taille d'ablation comme une fonction de la vitesse de changement de température de la section de rayonnement.

2. Appareil pour évaluer une taille d'ablation comme une fonction d'une information de température associée avec un applicateur d'énergie selon la revendication 1, comprenant en outre :
un moyen prévu pour, configuré pour et destiné à déterminer au moins un paramètre opérationnel associé avec la source de génération d'alimentation électrochirurgicale (216) sur la base d'au moins un paramètre de la taille d'ablation.

3. Appareil pour évaluer une taille d'ablation comme une fonction d'une information de température associée avec un applicateur d'énergie selon la revendication 2, dans lequel le moyen prévu pour, configuré pour et destiné à déterminer au moins un paramètre opérationnel associé avec la source de génération d'alimentation électrochirurgicale (216) est configuré pour sélectionner le paramètre opérationnel parmi le groupe consistant en une température, une impédance, une puissance, un courant, une tension, un mode de fonctionnement, et une durée d'application d'une énergie électromagnétique.

4. Appareil pour évaluer une taille d'ablation comme une fonction d'une information de température associée avec un applicateur d'énergie selon la revendication 2, dans lequel le moyen prévu pour, configuré pour et destiné à déterminer au moins un paramètre de la taille d'ablation est configuré pour sélectionner parmi le groupe consistant en un volume, une longueur, un diamètre, un diamètre minimum, un diamètre maximum et un centroïde.

5. Appareil pour évaluer une taille d'ablation comme une fonction d'une information de température associée avec un applicateur d'énergie selon la revendication 4, dans lequel un moyen prévu pour, configuré pour et destiné à causer une interruption de la délivrance d'énergie par l'intermédiaire de la section de rayonnement au tissu sur un intervalle de temps prédéterminé inclut un moyen configuré pour commander la source de génération d'alimentation électrochirurgicale (216).

6. Appareil selon la revendication 1, comprenant en outre :
un moyen prévu pour, configuré pour et destiné à surveiller le capteur de température sur un deuxième intervalle de temps prédéterminé pour obtenir une information de température associée avec la section de rayonnement ;
un moyen prévu pour, configuré pour et destiné à évaluer l'information de température pour estimer un volume d'ablation ; et
un moyen prévu pour, configuré pour et destiné à déterminer au moins un paramètre opérationnel associé avec la source de génération d'alimentation électrochirurgicale (216) sur la base d'au moins un paramètre du volume d'ablation estimé.

7. Appareil selon la revendication 6,
dans lequel le moyen prévu pour, configuré pour et destiné à surveiller le capteur de température sur un deuxième intervalle de temps prédéterminé pour obtenir une information de température associée avec la section de rayonnement inclut un moyen prévu pour, configuré pour et destiné à commander la source de génération d'alimentation électrochirurgicale (216) pour causer une interruption de la délivrance d'énergie par l'intermédiaire de la section de rayonnement au tissu sur la deuxième durée prédéterminée.

8. Appareil selon la revendication 6,
dans lequel l'au moins un paramètre opérationnel associé avec la source de génération d'alimentation électrochirurgicale (216) est choisi parmi le groupe consistant en une température, une impédance, une puissance, un courant, une tension, un mode de fonctionnement, et une durée d'application d'une énergie électromagnétique, et/ou dans lequel l'au moins un paramètre du volume d'ablation estimé est choisi parmi le groupe consistant en un volume, une longueur, un diamètre, un diamètre minimum, un diamètre maximum et un centroïde et/ou dans lequel le deuxième intervalle de temps prédéterminé fait suite au premier intervalle de temps prédéterminé.

9. Appareil selon la revendication 6, comprenant en outre :
un moyen prévu pour, configuré pour et destiné à délivrer une énergie à partir de la source de génération d'alimentation électrochirurgicale (216) par l'intermédiaire de la section de rayonnement au tissu sur un troisième intervalle de temps prédéterminé ;
un moyen prévu pour, configuré pour et destiné à surveiller le capteur de température sur un quatrième intervalle de temps prédéterminé pour obtenir une information de température supplémentaire associée avec la section de rayonnement ; et
un moyen prévu pour, configuré pour et destiné à évaluer l'information de température supplémentaire pour évaluer un changement du volume d'ablation estimé.

10. Appareil selon la revendication 9, comprenant en outre :
un moyen prévu pour, configuré pour et destiné à déterminer au moins un paramètre opérationnel associé avec la source de génération d'alimentation électrochirurgicale (216) sur la base du changement du volume d'ablation estimé.

11. Appareil selon la revendication 10,
dans lequel un moyen est prévu pour, configuré pour et destiné à prévoir le troisième intervalle de temps prédéterminé à la suite du deuxième intervalle de temps prédéterminé.

12. Appareil selon la revendication 10,
dans lequel un moyen est prévu pour, configuré pour et destiné à prévoir le quatrième intervalle de temps prédéterminé à la suite du troisième intervalle de temps prédéterminé.
